# EUROPEAN PATENT SPECIFICATION

(11) **EP 0 610 384 B1**
(45) Date of publication and mention of the grant of the patent: **03.09.1997**
(21) Application number: 92923131.4
(22) Date of filing: 30.10.1992
(51) Int. Cl.: C07C 63/70, C07C 51/363, C07C 205/58, C07C 201/12

(54) **PROCESS FOR THE PREPARATION OF FLUORINATED BENZOIC ACIDS**
VERFAHREN ZUR HERSTELLUNG VON FLUORINIERTEN BENZOESÄUREN
PROCEDE DE PREPARATION D'ACIDES BENZOIQUES FLUORES

(30) Priority: 31.10.1991 US 785851
(43) Date of publication of application: 17.08.1994
(60) Divisional application: 97101115.0
(73) Proprietor: ABBOTT LABORATORIES, Abbott Park, Illinois 60064-3500 (US)
(72) Inventor: WANG, Xiu, C., Park City, IL 60085 (US); KALARITIS, P., New Providence, NJ 07974 (US); CHANG, Michelle, L., Vernon Hill, IL 60061 (US)
(74) Representative: Modiano, Guido, Dr.-Ing.
(86) International application number: US9209300
(87) International publication number: WO9309077

(56) References cited:
- EP-A- 0 230 948
- EP-A- 0 303 291
- WO-A-94/04502
- DE-A- 3 917 904
- GB-A- 1 024 656
- DATABASE WPI Section Ch, Week 8908, Derwent Publications Ltd., London, GB; Class B05, AN 89-059218 & JP-A-1 013 037 (IHARA CHEM IND) 17 January 1989
- DATABASE WPI Section Ch, Week 8908, Derwent Publications Ltd., London, GB; Class B05, AN 89-059218 & JP-A-1 013 037

## Description

The present invention relates to the preparation of starting materials for use in the synthesis of quinolone antibacterial agents. More particularly, the invention relates to a process for preparing certain halo-substituted benzoic acids which may be employed in quinolone syntheses, as well as novel compounds useful in such a process.

### Background of the Invention

Substituted 1,4-dihydro-4-oxoquinoline-3-carboxylic acid derivatives (hereinafter quinolones) are known to be effective antibacterial agents (see, for example, United States Patent No. 4,730,000, issued March 8, 1988 to Chu). Halo-substituted benzoic acids and their corresponding esters and acetophenones are useful as starting materials in the synthesis of such quinolones, as disclosed in the published European patent application of Kumai *et al.,* No. 0 303 291, published February 15, 1989.

In particular, 2-chloro-4,5-difluorobenzoic acid (CDFBA), 2,4,5-trifluorobenzoic acid (TFBA), and their respective analogous acetophenones are advantageous starting materials for quinolone synthesis. Known methods of preparing these compounds, however, suffer from a number of drawbacks, including complex chemistry requiring specialized equipment; expensive or hard-to-obtain starting materials; materials hazards such as those associated with the use and decomposition of diazonium salts; and reactions having commercially undesirable selectivities and/or yields. There is therefore a continuing need for an improved process for preparing the above intermediates which overcomes some or all of these disadvantages.

### Summary of the Invention

Accordingly, a new process is disclosed for the preparation of CDFBA and TFBA from inexpensive and readily available starting materials. In one aspect of the present invention is disclosed a method for preparing a compound having the formula 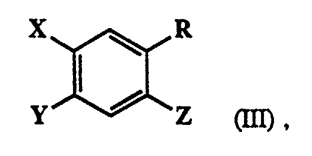 wherein X is chloro or fluoro; one of Y and Z is chloro and the other of Y and Z is nitro; and R is a radical selected from the group consisting of -CCl₃, -CH₂NO₂, -CH(NO₂)R¹, -CH(CO₂R¹)₂, -CH(C(O)R²)₂, -CH(CN)CO₂R¹, -CH(CO₂R¹)COR² and -COR², where R¹ is alkyl or arylalkyl and R² is alkyl, aryl or arylalkyl and, where appearing more than once in such a radical, R¹ and R² may be the same or different at each occurrence. The method comprises reacting a nitrobenzene having the formula 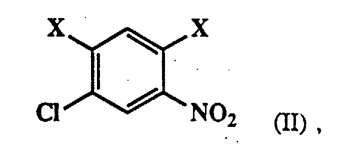 with an appropriate carbanion to form said compound (III). Such a carbanion may be generated by reacting a base with a nucleophile such as one selected from the group consisting of nitroalkanes, enamines, malonates, beta-ketoesters, cyanoacetates, malononitriles and beta-diketones. Bases which are suitable for this reaction include, for example, those selected from the group consisting of amines, amidines, hydroxides, alkoxides, hydrides, carbonates and bicarbonates. A preferred embodiment of the method is one in which the base is 1,8-diazabicyclo[5.4.0]undec-7-ene (DBU) or 1,1,3,3-tetramethylguanidine, forming a substituted nitrobenzene product (III) in which R is -CH₂NO₂ or -CH(NO₂)CH₃, X is fluoro, Y is nitro; and Z is chloro.

Also comprised by the present invention are novel synthetic intermediates which may be prepared according to the above inventive methods. Included are compounds having the formula 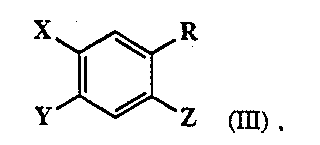 wherein, as before, X is chloro or fluoro; one of Y and Z is chloro and the other is nitro; and R is selected from the group consisting of -CCl₃, -CH₂NO₂, -CH(NO₂)R¹, -CH(CO₂R¹)₂, -CH(C(O)R²)₂, -CH(CN)CO₂R¹, -CH(CO₂R¹)COR² and -COR², where R¹ and R² are as previously defined.

### Detailed Description of the Invention

The methods and compounds of the present invention are herein described using certain terms which, except where otherwise indicated, are accorded the following definitions:

The term "alkoxide" refers to a compound of the formula R³OM or (R³O)₂M, where R³ is alkyl as defined below and M is a suitable cation such as lithium, sodium, potassium or magnesium.

The term "alkyl" refers to a straight- or branched-chain, saturated hydrocarbon radical of one to ten carbon atoms including, but not limited to, methyl, ethyl, propyl, isopropyl, *n*-butyl, *sec-*butyl*, tert*-butyl and the like.

The term "amidine" refers to a compound of the formula R⁴C(NR⁵R⁶)=NR⁷ where R⁵, R⁶ and R⁷ are independently alkyl and R⁴ is selected from amino and alkyl, or where either or both of R⁵ and R⁶ taken together with either or both of R⁴ and R⁷ form a group having the formula -(CH₂)ₘ- where m is two to six including, but not limited to, DBU, 1,5-diazabicyclo[4.3.0]non-5-ene (DBN), guanidines such as 1,1,3,3-tetramethyl-guanidine or 2-*t*-butyl-1,1,3,3-tetramethylguanidine and the like.

The term "amine" refers to a tertiary amine of the formula N(R⁸)₃ or a tertiary diamine of the formula N(R⁹)₃N, where R⁸ is alkyl of from two to ten carbons or arylalkyl having an alkyl component of from two to ten carbons and R⁹ is a group of the formula -(CH₂)ₙ- where n is two to four, including, but not limited to, triethylamine, triethylenediamine and the like.

The term "aryl" refers to a cyclic or fused bicyclic, aromatic hydrocarbon radical such as phenyl or naphthyl.

The term "arylalkyl" refers to an aryl radical linked to the parent molecule via an alkyl group including, but not limited to, benzyl, phenylethyl, phenylbutyl, naphthylmethyl, naphthylpentyl and the like.

The term "beta-diketone" refers to a compound of the formula R¹¹C(O)CH(R¹⁰)C(O)R¹², where R¹⁰ is selected from hydrogen, alkyl, aryl and arylalkyl and R¹¹ and R¹² are independently alkyl, aryl or arylalkyl.

The term "beta-ketoester" refers to a compound of the formula R¹¹C(O)CH(R¹³)CO₂R²¹, where R¹³ is selected from hydrogen, alkyl and arylalkyl, R¹¹ is alkyl, aryl or arylalkyl, and R²¹ is alkyl or arylalkyl.

The term "bicarbonate" refers to a compound of the formula M¹HCO₃, where M¹ is a suitable cation such as lithium, sodium or potassium.

The term "carbonate" refers to a compound of the formula (M¹)₂CO₃ where M¹ is a suitable monovalent cation such as lithium, sodium or potassium, or of the formula M²CO₃ where M² is a divalent cation such as magnesium or calcium.

The term "cyanoacetate" refers to a compound of the formula CH(CN)(R¹⁴)CO₂R¹⁵, where R¹⁴ and R¹⁵ are independently selected from hydrogen, alkyl and arylalkyl.

The term "enamine" refers to a compound of the formula C(NR¹⁶R¹⁷)(R¹⁴)=CHR¹⁵, where R¹⁶ and R¹⁷ are independently alkyl of one to ten carbons and R¹⁴ and R¹⁵ are independently selected from hydrogen, alkyl and arylalkyl.

The term "fluoride" refers to a compound of the formula M³F, where M³ is a suitable cation such as lithium, sodium, potassium, cesium or alkylammonium.

The term "hydride" refers to a compound of the formula M¹H, where M¹ is a suitable cation such as lithium, sodium or potassium.

The term "hydroxide" refers to a compound of the formula M⁴OH, where M⁴ is a suitable cation such as lithium, sodium, potassium, magnesium or alkylammonium.

The term "malonate" refers to a compound of the formula CH(R¹³)(CO₂R¹⁸)₂, where R¹³ and R¹⁸ are selected from hydrogen, alkyl and arylalkyl.

The term "malononitrile" refers to a compound of the formula CH(R¹⁰)(CN)₂, where R¹⁰ is selected from hydrogen, alkyl, aryl and arylalkyl.

The term "nitroalkane" refers to a compound having the formula CH(R¹⁹)(R²⁰)NO₂, where R¹⁹ and R²⁰ are independently selected from hydrogen, alkyl and aryl.

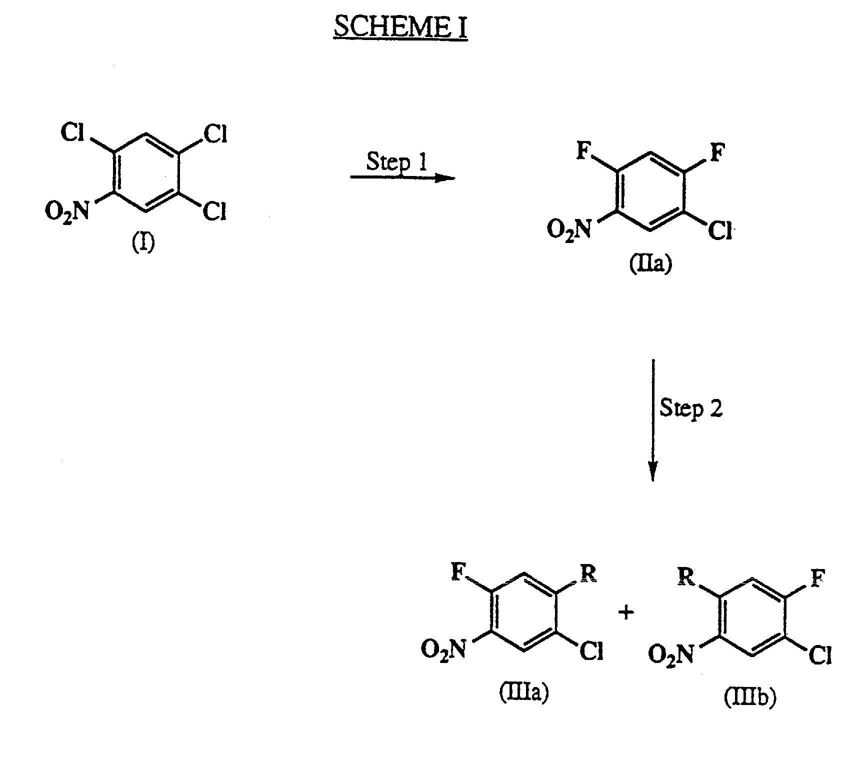

The present invention will be better understood in connection with the preceding reaction scheme. In Scheme I, 2,4,5-trichloronitrobenzene (I) is converted to 5-chloro-2,4-difluoronitrobenzene (IIa) in a fluorination reaction (reaction step 1) using a fluorinating reagent such as potassium fluoride. Upon exposure to an anion of a nucleophile such as nitromethane or malonate, compound (IIa) undergoes a nucleophilic substitution reaction (step 2) to produce isomeric nitrobenzene derivatives (IIIa) and (IIIb).

The above procedures may be carried out using a variety of reagents and reaction conditions. In the substitution reactions of step 2 which are demonstrated in Examples 2, 7-10 and 14-18 below, a variety of solvents are suitable including common protic or aprotic polar solvents such as DMF, dimethyl sulfoxide (DMSO), dioxane, pyridine, THF, N,N-dimethylacetamide (DMAC), and two- to five-carbon alcohols, as well as mixtures thereof such as DMSO/water. Alternatively, the nucleophilic substitutions of these steps may be performed neat in the presence of a phase-transfer catalyst, as for example tris(3,6-dioxaheptyl)amine (TDA), tricaprylylmethylammonium chloride, tetrabutylammonium fluoride (TBAF) or tetrabutylammonium chloride (TBAC).

The foregoing methods and compounds of the present invention may be better understood by reference to the following Examples, which are provided for illustration and are not intended as a limitation upon the present invention.

### Example 1

### 5-Chloro-2,4-difluoronitrobenzene (2)

200 g (0.883 mol) of 2,4,5-trichloronitrobenzene and 128.4 g (2.21 mol) of KF in 500 ml of tetramethylenesulfone were reacted at 200°C under nitrogen for 3.5 hours. To the mixture was added 500 ml of ethyl acetate and the precipitate was filtered. The filtrate was washed with brine, dried over magnesium sulfate and concentrated. Distillation at 85-90°C/0.9 mm afforded pure compound **2** with more than 70% yield.

¹H-NMR from CDCl₃ (δ ppm): 7.17(dd, 1H, J=8Hz, 9Hz), 8.26 (dd, 1H, J=7.5Hz, 7.5Hz).

MS(*m/z*): 193 (M⁺).

### Example 2

### 5-Chloro-2-fluoro-4-(nitromethyl)nitrobenzene (3) and 5-Chloro-4-fluoro-2-(nitromethyl)nitrobenzene (4)

DBU (16.5 g, 0.11 mol) in 20 ml of ethyl acetate was cooled in an ice-bath and treated with nitromethane (3.1 ml, 0.057 mol). The solution was stirred under nitrogen for 10 min and 10.0 g of compound **2** (0.052 mol, from example 1) in 20 ml of ethyl acetate was added dropwise at the same temperature. The dark red mixture was stirred for 2 hours and then warmed up to room temperature. The mixture was treated with 10% HCl (10 ml) and extracted with ethyl acetate. The ethyl acetate layer was washed with brine, dried over sodium sulfate, filtered, and concentrated under reduced pressure to give the product (10.9 g, 89.9%). The product consisted of compounds **3** and **4** in a ratio of 2:1 based on NMR analysis. The two isomers were separated by column chromatography (silica gel, 10% ethyl acetate/hexanes).

Compound **3**: yellowish crystals, mp 70-72°C. ¹H-NMR from CDCl₃ (δ ppm): 5.64 (s, 2H), 7.47 (d, 1H, J=10.5Hz), 8.22 (d, 1H, J=7Hz).

Compound **4**: ¹H-NMR from CDCl₃ (δ ppm): 5.81 (s, 2H), 7.31 (d, 1H, J=9Hz), 8.44 (d, 1H, J=6Hz).

MS (*m/z*): 234 (M⁺).

### Example 3

### Dimethyl-2-(2-chloro-5-fluoro-4-nitrophenyl)malonate (9) and Dimethyl 2-(4-chloro-5-fluoro-2-nitrophenyl)malonate (10)

### Diethyl 2-(2-chloro-5-fluoro-4-nitrophenyl)malonate (9a) and Diethyl 2-(4-chloro-5-fluoro-2-nitrophenyl)malonate (10a)

Variation 1: The preparations of compounds **9** and **9a** and their respective isomers **10** and **10a** were carried out in the same manner as in Example 2 using dimethyl and diethyl malonate, respectively, in place of nitromethane. The products were isolated in quantitative yield. In each reaction, a 1.2:1 ratio mixture of the 2-chloro-5-fluoro-4-nitrophenyl and 4-chloro-5-fluoro-2-nitrophenyl malonates was formed, based on NMR analysis. The isomeric products were separated by column chromatography (silica gel, 10% EtOAc/hexanes).

Variation 2: To suspensions of lithium hydroxide monohydrate (47.9 g, 1.14 mol) in DMSO (100ml) at 15°C were added the appropriate dialkyl malonate (86.4 ml, 0.57 mol). The mixtures were stirred for 15 min under nitrogen, and a solution of compound **2** (from Example 1, 100 g, 0.52 mol) in 50 ml of DMSO added dropwise. The dark red mixtures were stirred under nitrogen at room temperature for 3 hours, quenched with 10% HCl, and extracted with ethyl acetate. The ethyl acetate layers were washed with brine and dried over magnesium sulfate. The solvent was removed under reduced pressure to give the pure products (quantitative yield), consisting in each case of the 2-chloro-5-fluoro-4-nitrophenyl and 4-chloro-5-fluoro-2-nitrophenyl malonates in a ratio of 1.2:1 based on NMR analysis.

Compound **9**: mp 51-51°C. ¹H-NMR from CDCl₃ (δ ppm): 3.82 (s,6 H), 5.26 (s, 1H), 7.59 (d, 1H, J=10Hz), 8.15 (d, 1H, J=7Hz).

Compound **10**: ¹H-NMR from CDCl₃ (δ ppm): 3.82 (s.6 H), 5.37 (s, 1H), 7.48 (d, 1H, J=10Hz), 8.22 (d, 1H, J=7Hz).

MS (*m/z*): 323 ([M+NH₄]⁺).

Compound **9a**: ¹H-NMR from CDCl₃ (δ ppm): 1.30 (t, 6H, J=7Hz), 4.29 (q, 4H), 5.22 (s, 1H), 7.60 (d, 1H, J=11Hz), 8.15 (d, 1H, J=7Hz).

Compound **10a:** ¹H-NMR from CDCl₃ (δ ppm): 1.31 (t, 6H, J=7Hz), 4.29 (q, 4H), 5.32 (s, 1H), 7.40 (d, 1H, J=9Hz), 8.22 (d, 1H, J=7Hz).

MS (*m/z*): 351 ([M+NH₄]⁺).

### Example 4

### Methyl 2-(2-chloro-5-fluoro-4-nitrophenyl)cyanoacetate (11) and Methyl 2-(4-chloro-5-fluoro-2-nitrophenyl)cyanoacetate (12)

The preparation of compounds **11** and **12** was carried out in a manner analogous to that of Example 2 using 1 g (5.2 mmol) of compound **2** (from Example 1) and methyl cyanoacetate. The product consisted of **11** and **12** in a ratio of 1:2 with a total yield of 100%. The two isomers were separated by column chromatography (silica gel, 10% EtOAc/hexanes).

Compound **11**: ¹H-NMR from CDCl₃ (δ ppm): 3.90 (s, 3H), 5.21 (s, 1H), 7.62 (d, 1H, J = 11 Hz), 8.20 (d, 1H, J = 7 Hz).

Compound **12:** off-white solid, mp 92.5-94°C. ¹H-NMR from CDCl₃ (δ ppm): 3.90 (s, 3H), 5.70 (s, 1H), 7.61 (d, 1H, J = 9 Hz), 8.39 (d, 1H, J = 7 Hz).

MS (*m/z*): 290 ([M+NH₄]⁺).

### Example 5

### Ethyl 2-(2-Chloro-5-fluoro-4-nitrophenyl)acetoacetate (13) and Ethyl 2-(4-chloro-5-fluoro-2-nitrophenyl)acetoacetate (14)

The preparation of compounds **13** and **14** was conducted in the same manner as in Example 7 with 0.5 g of compound **2** (from Example 1) and ethyl acetoacetate. The product consisted of compounds **13** and **14** in a ratio of 1:1 with a total yield of 0.7 g (89%). The two isomeric products were separated by column chromatography (silica gel, 10% EtOAc/hexanes).

Compound **13**: ¹H-NMR from CDCl₃ (δ ppm): 1.20 (t, 3H, J = 7.5 Hz, 7.5 Hz), 1.84 (s, 3 H), 4.24 (q, 2H), 7.29 (d, 1H, J = 11 Hz), 8.16 (d, 1H, J = 7 Hz), 13.15 (s, 1H).

Compound **14**: ¹H-NMR from CDCl₃ (δ ppm): 1.12 (t, 3H, J = 7 Hz), 1.90 (s, 3H), 4.16 (q, 2H), 7.09(d, 1H, J = 8Hz), 8.16 (d, 1H, J = 7 Hz), 13.04 (s, 1H).

MS (*m/z*): 321 ([M+NH₄]⁺).

### Example 6

### 5-Chloro-2-fluoro-4-(trichloromethyl)nitrobenzene (15) and 5-Chloro-4-fluoro-2-(trichloromethyl)nitrobenzene (16)

The preparation of compounds **15** and **16** was carried out as in Example 2 using chloroform and compound **2**. The product consisted of compounds **15** and **16** in a ratio of 1:1 with a total yield of 44%.

Compound **15**: ¹H-NMR from CDCl₃ (δ ppm): 6.91 (d, 1H, J=12Hz), 8.12 (d, 1H, J=7Hz).

Compound **16**: ¹H-NMR from CDCl₃ (δ ppm): 6.32 (d, 1H, J=13Hz), 8.29 (d, 1H, J=7Hz).

### Example 7

### 2,5-Dichloro-4-(1-nitroethyl)nitrobenzene (17) and 4,5-Dichloro-2-(1-nitroethyl)nitrobenzene (18)

To a solution of 75.5 g of DBU (0.486 mol) in 350 ml of ethyl acetate cooled in an ice bath were sequentially added dropwise 18.2 g (0.243 mol) of nitroethane and 50 g (0.221 mol) of 2,4,5-trichloronitrobenzene (compound 1) in 100 ml of ethyl acetate. The resultant mixture was stirred under nitrogen at room temperature for 2 days. The mixture was acidified with 10% HCl and extracted with ethyl acetate. The ethyl acetate solution was washed with brine, dried over magnesium sulfate, and concentrated. The product obtained consisted of compounds **17** and **18** in a ratio of 1:3 based on NMR analysis (50.3 g, 86%). The two isomers were separated by chromatography (silica gel, 10% EtOAc/hexanes).

2,5-Dichloro-4-(1-nitroethyl)nitrobenzene (**17**): ¹H-NMR from CDCl₃ (δ ppm): 1.97 (d, 3H, J=7Hz), 6.06 (q, 1H), 7.67 (s, 1H), 8.01(s, 1H).

4,5-Dichloro-2-(1-nitroethyl)nitrobenzene (**18**): mp 48-49°C. ¹H-NMR from CDCl₃ (δ ppm): 2.01(d, 3H, J=7Hz), 6.25 (q, 1H), 7.71(s, 1H), 8.23 (s, 1H).

MS(*m/z*): 282([M+NH₄]⁺).

### Example 8

### Dimethyl 2-(4,5-dichloro-2-nitrophenyl)malonate (19) and Dimethyl 2-(2,5-dichloro-4-nitrophenyl)malonate (20)

The preparation of compounds **19** and **20** was carried out as in Example 2 using dimethyl malonate in place of nitromethane. The yellowish crystalline product consisted of compounds **19** and **20** in a ratio of 2.5:1 with a total yield of 100%.

Compound **19**: ¹H-NMR from CDCl₃(δ ppm): 3.76 (s, 6H), 5.24 (s, lH), 7.78 (s, 1H), 7.97 (s, 1H).

Compound **20:** ¹H-NMR from CDCl₃(δ ppm): 3.83 (s, 6H), 5.31 (s, 1H), 7.65 (s, 1H), 8.21(s, 12H).

MS (*m/z*): 339 ([M+NH₄]⁺).

### Example 9

### Methyl 2-(2,5-Dichloro-4-nitrophenyl)cyanoacetate (21) and Methyl 2-(4,5-Dichloro-2-nitrophenyl)cyanoacetate (22)

The preparation of compounds **21** and **22** was carried out as in Example 2 using methyl cyanoacetate in place of nitromethane. The product consisted of compounds **21** and **22** in a ratio of 1:3 with a total yield of 93%. The two isomers were separated by column chromatography (silica gel, 8% EtOAc/hexanes).

Compound **21**: ¹H-NMR from CDCl₃ (δ ppm): 3.90 (s, 3H), 5.20 (s, 1H), 7.82 (s, 1H), 8.01 (s, 1H).

Compound **22:** white solid, mp 98.7-99.8°C. ¹H-NMR from CDCl₃ (δ ppm): 3.90 (s, 3H), 5.68 (s, 1H), 7.89 (s, 1H), 8.48 (s, 1H).

MS (*m/z*): 306 ([M+NH₄]⁺).

### Example 10

### Ethyl 2-(2,5-Dichloro-4-nitrophenyl)acetoacetate (23) and Ethyl 2-(4,5-Dichloro-2-nitrophenyl)acetoacetate (24)

The preparation of compounds **23** and **24** was carried out as in Example 7 using ethyl acetoacetate in place of nitromethane. The product consisted of compounds **23** and **24** in a ratio of 1:3 with a total yield of 90%. The two isomers were separated by column chromatography (silica gel, 8% ethyl acetate/hexanes).

Compound **23**: ¹H-NMR from CDCl₃ (δ ppm): 1.20 (t, 3H, J=7.5Hz), 1.85 (s, 3H), 4.24 (m, 2H), 7.42 (s, 1H), 8.0 (s, 1H), 13.15 (s, 1H).

Compound **24**: ¹H-NMR from CDCl₃ (δ ppm): 1.13 (t, 3H, J=7.5Hz), 1.91 (s, 3H), 4.22 (m, 2H), 7.40 (s, 1H), 8.14 (s, 1H), 13.05 (s, 1H).

MS (*m/z*): 337 ([M+NH₄]⁺).

### Example 11

### 2-Chloro-5-fluoro-4-(1-nitroethyl)nitrobenzene (27) and 4-Chloro-5-fluoro-2-(1-nitroethyl)nitrobenzene (28)

A solution of 1.4 ml ( 10.2 mmol) of DBU in 2 ml of ethyl acetate was cooled to 0°C and treated sequentially with 0.38 ml (5.3 mmol) of nitroethane and 1.0 g (5.2 mmol) of compound 2 in 3 ml of ethyl acetate. The dark-red solution was stirred for 1 hour (0°C-20°C) and then acidified with 10% HCl. The mixture was extracted with ethyl acetate, and the ethyl acetate solution dried over sodium sulfate and concentrated to give 1.1 g (84%) of pure compounds **27** and **28** in a ratio of 2:1. The two isomeric products were separated by column chromatography (silica gel, 10% EtOAc/hexanes).

Compound **27:** ¹H-NMR from CDCl₃ (δ ppm): 1.96 (d, 3H, J=7Hz), 6.08 (q, 1H), 7.46 (d, 1H, J=11Hz), 8.20 (d, 1H, J=6.5Hz).

Compound **28:** ¹H-NMR from CDCl₃ (δ ppm): 2.0 (d, 3H, J=7Hz), 6.28 (q, 1H), 7.42 (d, 1H, J=10Hz), 8.25 (d, 1H, J=6.5Hz).

MS (*m/z*): 248 (M⁺).

## Claims

1. A method for preparing a compound having the formula 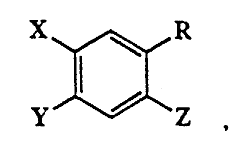 wherein X is chloro or fluoro; one of Y and Z is chloro and the other is nitro; and R is a radical selected from the group consisting of -CCl₃, -CH₂NO₂, -CH(NO₂)R¹, -CH(CO₂R¹)₂, -CH(C(O)R²)₂, -CH(CN)CO₂R¹, -CH(CO₂R¹)COR² and -COR² when R¹ is alkyl or arylalkyl and R² is alkyl, aryl or arylalkyl and, when appearing more than once in such a radical, R¹ and R² may be the same or different at each occurrence, wherein the term alkyl refers to a straight-or branched-chain saturated hydrocarbon radical of one to ten carbon atoms and the term aryl refers to a cyclic or fused bicyclic aromatic hydrocarbon radical, said method comprising reacting a nitrobenzene having the formula 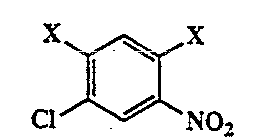 with an appropriate carbanion to form said compound.

2. The method according to Claim 1 wherein said carbanion is generated by reacting a base with a nucleophile-selected from the group consisting of chloroform, nitroalkanes of formula CH(R¹⁹)(R²⁰)NO₂, enamines of formula C(NR¹⁶R¹⁷)(R¹⁴)=CHR¹⁵, malonates of formula CH(R¹³)(CO₂R¹⁸)₂, beta-ketoesters of formula R¹¹C(O)CH(R¹³)CO₂R²¹, cyanoacetates of formula CH(CN)(R¹⁴)CO₂R¹⁵, malononitriles of formula CH(R¹⁰)(CN)₂, and beta-diketones of formula R¹¹C(O)CH(R¹⁰)C(O)R¹², when R¹⁹ and R²⁰ are independently selected from the group consisting of hydrogen, alkyl and aryl; R¹⁴ and R¹⁵ are independently selected from the group consisting of hydrogen, alkyl and arylalkyl; R¹⁶ and R¹⁷ are independently alkyl; R¹³ and R¹⁸ are independently selected from the group consisting of hydrogen, alkyl and arylalkyl; R¹¹ and R¹² are independently selected from the group consisting of alkyl, aryl and arylalkyl; R¹⁰ is selected from the group consisting of hydrogen, alkyl, aryl and arylalkyl; and R²¹ is selected from the group consisting of alkyl or arylalkyl, wherein the term alkyl refers to a straight- or branched-chain saturated hydrocarbon radical of one to ten carbon atoms and the term aryl refers to a cyclic or fused bicyclic aromatic hydrocarbon radical.

3. The method according to Claim 2 wherein said base is selected from the group consisting of tertiary amines of the formula N(R⁸)₃ or tertiary diamines of the formula N(R⁹)₃N where R⁸ is alkyl of from two to ten carbons or arylalkyl having an alkyl component of from two to ten carbons and R⁹ is a group of the formula -(CH₂)ₙ- where n is two to four, amidines of the formula R⁴C(NR⁵R⁶)=NR⁷ where R⁵, R⁶ and R⁷ are independently alkyl of one to ten carbons and R⁴ is selected from amino and alkyl of one to ten carbons or where either or both of R⁵ and R⁶ taken together with either or both of R⁴ and R⁷ form a group having the formula -(CH₂)ₘ- where m is two to six, hydroxides of the formula M⁴OH where M⁴ is a suitable cation, alkoxides of the formula R³OM or (R³O)₂M where R³ is alkyl of one to ten carbons and M is a suitable cation, hydrides of the formula M¹H where M¹ is a suitable cation, carbonates of the formula (M¹)₂CO₃ where M¹ is a suitable monovalent cation or of the formula M²CO₃ where M² is a divalent cation, and bicarbonates of the formula M¹HCO₃ where M¹ is a suitable cation.

4. The method according to claim 3 wherein said base is 1,8-diazabicyclo[5.4.0]undec-7-ene or 1,1,3,3-tetramethylguanidine; R is -CH₂NO₂ or -CH(NO₂)CH₃; X is fluoro; Y is nitro; and Z is chloro.

5. A compound having the formula 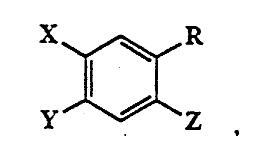 wherein X is chloro or fluoro; one of Y and Z is chloro and the other is nitro; and R is a radical selected from the group consisting of -CCl₃, -CH₂NO₂, -CH(NO₂)R¹, -CH(CO₂R¹)₂, -CH(C(O)R²)₂, -CH(CN)CO₂R¹ and -CH(CO₂R¹)COR² where R¹ is alkyl or arylalkyl and R² is alkyl, aryl or arylalkyl and, when appearing more than once in such a radical, R¹ and R² may be the same or different at each occurrence, wherein the term alkyl refers to a straight- or branched-chain saturated hydrocarbon radical of one to ten carbon atoms and the term aryl refers to a cyclic or fused bicylic aromatic hydrocarbon radical.

## Patentansprüche

1. Verfahren zur Herstellung einer Verbindung nach folgender Formel: 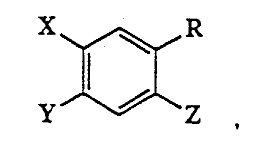 worin X gleich Chlor oder Fluor ist; worin einer von Y und Z gleich Chlor ist und der andere gleich Nitro; und worin R ein Radikal ist, das aus der Gruppe gewählt ist, die aus -CCl₃, -CH₂NO₂, -CH(NO₂)R¹, -CH(CO₂R¹)₂, -CH(C(O)R²)₂, -CH(CN)CO₂R¹, -CH(CO₂R¹)COR² und -COR² besteht, worin R¹ gleich Alkyl oder Arylalkyl ist und R² gleich Alkyl, Aryl oder Arylalkyl und worin, wo sie mehr als einmal in solch einem Radikal auftreten, R¹ und R² bei jedem Auftreten gleich oder verschieden sein können, worin der Ausdruck Alkyl ein geradkettiges oder verzweigtkettiges gesättigtes Kohlenwasserstoffradikal mit ein bis zehn Kohlenstoffatomen bezeichnet, und der Ausdruck Aryl ein cyclisches oder anneliertes bicyclisches aromatisches Kohlenwasserstoffradikal bezeichnet, wobei das Verfahren die Umsetzung eines Nitrobenzols nach folgender Formel: 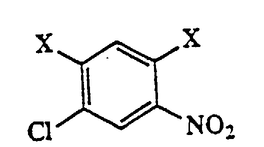 mit einem geeigneten Carbanion unter Ausbildung der Verbindung umfaßt.

2. Verfahren nach Anspruch 1 , wobei das Carbanion durch Umsetzung einer Base mit einem Nukleophil erzeugt wird, das aus der Gruppe gewählt ist, die aus Chloroform, aus Nitroalkanen der Formel CH(R¹⁹)(R²⁰)NO₂, aus Enaminen der Formel C(NR¹⁶R¹⁷)(R¹⁴)=CHR¹⁵, aus Malonaten der Formel CH(R¹³)(CO₂R¹⁸)₂, aus beta-Ketoestern der Formel R¹¹C(O)CH(R¹³)CO₂R²¹, aus Cyanoacetaten der Formel CH(CN)(R¹⁴)CO₂R¹⁵, aus Malononitrilen der Formel CH(R¹⁰)(CN)₂ und aus beta-Diketonen der Formel R¹¹C(O)CH(R¹⁰)C(O)R¹² besteht, wobei R¹⁹ und R²⁰ unabhängig aus der Gruppe gewählt sind, die aus Wasserstoff, Alkyl und Aryl besteht; wobei R¹⁴ und R¹⁵ unabhängig aus der Gruppe gewählt sind, die aus Wasserstoff, Alkyl und Arylalkyl besteht; wobei R¹⁶ und R¹⁷ unabhängig gleich Alkyl sind; wobei R¹³ und R¹⁸ unabhängig aus der Gruppe gewählt sind, die aus Wasserstoff, Alkyl und Arylalkyl besteht; wobei R¹¹ und R¹² unabhängig aus der Gruppe gewählt sind, die aus Alkyl, Aryl und Arylalkyl besteht; wobei R¹⁰ aus der Gruppe gewählt ist, die aus Wasserstoff, Alkyl, Aryl und Arylalkyl besteht; und wobei R²¹ aus der Gruppe gewählt ist, die aus Alkyl oder Arylalkyl besteht, wobei der Ausdruck Alkyl ein geradkettiges oder verzweigtkettiges gesättigtes Kohlenwasserstoffradikal mit ein bis zehn Kohlenstoffatomen bezeichnet, und wobei der Ausdruck Aryl ein cyclisches oder anneliertes bicyclisches aromatisches Kohlenwasserstoffradikal bezeichnet.

3. Verfahren nach Anspruch 2, wobei die Base aus der Gruppe gewählt ist, die aus tertiären Aminen der Formel N(R⁸)₃ oder aus tertiären Diaminen der Formel N(R⁹)₃N, wobei R⁸ gleich Alkyl mit zwei bis zehn Kohlenstoffen oder gleich Arylalkyl mit einem Alkylbestandteil mit zwei bis zehn Kohlenstoffen ist, und wobei R⁹ eine Gruppe nach Formel -(CH₂)ₙ- ist, wobei n gleich zwei bis vier ist, aus Amidinen der Formel R⁴C(NR⁵R⁶)=NR⁷, wobei R⁵, R⁶ und R⁷ unabhängig gleich Alkyl mit ein bis zehn Kohlenstoffen sind, und wobei R⁴ aus Amino und Alkyl mit ein bis zehn Kohlenstoffen gewählt ist, oder wobei einer oder beide von R⁵ und R⁶ zusammengenommen mit einem oder beiden von R⁴ und R⁷ eine Gruppe mit der Formel -(CH₂)ₘ- ausbilden, wobei m gleich zwei bis sechs ist, aus Hydroxiden der Formel M⁴OH, wobei M⁴ ein geeignetes Kation ist, aus Alkoxiden der Formel R³OM oder (R³O)₂M, wobei R³ gleich Alkyl mit ein bis zehn Kohlenstoffen ist, und wobei M gleich einem geeigneten Kation ist, aus Hydriden der Formel M¹H, wobei M¹ ein geeignetes Kation ist, aus Carbonaten der Formel (M¹)₂CO₃, wobei M¹ ein geeignetes monovalentes Kation ist, oder der Formel M²CO₃, wobei M² ein divalentes Kation ist, und aus Bicarbonaten der Formel M¹HCO₃, wobei M¹ ein geeignetes Kation ist, besteht.

4. Verfahren nach Anspruch 3, wobei die Base gleich 1,8-Diazabicyclo[5.4.0]undec-7-en ist oder gleich 1,1,3,3-Tetramethylguanidin; wobei R gleich -CH₂NO₂ oder -CH(NO₂)CH₃ ist; wobei X gleich Fluor ist; wobei Y gleich Nitro ist; und wobei Z gleich Chlor ist.

5. Verbindung nach folgender Formel: 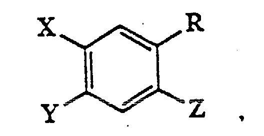 worin X gleich Chlor oder Fluor ist; worin einer von Y und Z gleich Chlor ist und der andere gleich Nitro; und worin R ein Radikal ist, das aus der Gruppe gewählt ist, die aus -CCl₃, -CH₂NO₂, -CH(NO₂)R¹, -CH(CO₂R¹)₂, -CH(C(O)R²)₂, -CH(CN)CO₂R¹ und -CH(CO₂R¹)COR² besteht, worin R¹ gleich Alkyl oder Arylalkyl ist und R² gleich Alkyl, Aryl oder Arylalkyl ist und worin, wo sie mehr als einmal in solch einem Radikal auftreten, R¹ und R² bei jedem Auftreten gleich oder verschieden sein können, worin der Ausdruck Alkyl ein geradkettiges oder verzweigtes gesättigtes Kohlenwasserstoffradikal mit ein bis zehn Kohlenstoffatomen bezeichnet, und worin der Ausdruck Aryl ein cyclisches oder anneliertes bicyclisches aromatisches Kohlenwasserstoffradikal bezeichnet.

## Revendications

1. Méthode de préparation d'un composé de formule 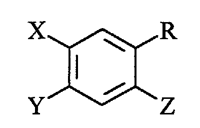 dans laquelle X est chloro ou fluoro, un parmi Y et Z est chloro et l'autre est nitro et R est un radical choisi dans le groupe constitué par -CCl₃, -CH₂NO₂, -CH(NO₂)R¹, -CH(CO₂R¹)₂, -CH(C(O)R²)₂, -CH(CN)CO₂R¹, -CH(CO₂R¹)COR², et -COR², dans lesquels R¹ représente un alkyle ou un arylalkyle et R² représente un alkyle, un aryle ou un arylalkyle et, où, s'ils apparaissent plus d'une fois dans un tel radical, R¹ et R² peuvent être à chaque fois, identiques ou différents, le terme alkyle représentant un radical hydrocarboné saturé à chaîne droite ou ramifiée contenant un à dix atomes de carbone et le terme aryle représentant un radical hydrocarboné aromatique cyclique ou bicyclique condensé, ladite méthode comprenant la réaction d'un nitrobenzène de formule 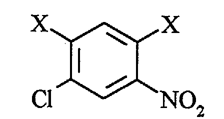 avec un carbanion approprié pour former ledit composé.

2. Méthode selon la revendication 1, dans laquelle ledit carbanion est généré par la réaction entre une base et un nucléophile choisi dans le groupe constitué par le chloroforme, les nitroalcanes de formule CH(R¹⁹)(R²⁰)NO₂, les énamines de formule C(NR¹⁶R¹⁷)(R¹⁴)=CHR¹⁵, les malonates de formule CH(R¹³)(CO₂R¹⁸)₂, les béta-cétoesters de formule R¹¹C(O)CH(R¹³)CO₂R²¹, les cyanoacétates de formule CH(CN)(R¹⁴)CO₂R¹⁵, les malononitriles de formule CH(R¹⁰)(CN)₂ et les béta-dicétones de formule R¹¹C(O)CH(R¹⁰)C(O)R¹², formules dans lesquelles R¹⁹ et R²⁰ sont, chacun de façon indépendante, choisis dans le groupe constitué par l'hydrogène, un alkyle et un aryle, R¹⁴ et R¹⁵ sont, chacun de façon indépendante, choisis dans le groupe constitué par l'hydrogène, un alkyle et un arylalkyle, R¹⁶ et R¹⁷ sont, chacun de façon indépendante, un alkyle, R¹³ et R¹⁸ sont, chacun de façon indépendante, choisis dans le groupe constitué par l'hydrogène, un alkyle et un arylalkyle, R¹¹ et R¹² sont, chacun de façon indépendante, choisis dans le groupe constitué par un alkyle, un aryle et un arylalkyle, R¹⁰ est choisi dans le groupe constitué par l'hydrogène, un alkyle, un aryle et un arylalkyle et R²¹ est choisi dans le groupe constitué par un alkyle et un arylalkyle, le terme alkyle représentant un radical hydrocarboné saturé à chaîne droite ou ramifiée contenant un à dix atomes de carbone et le terme aryle représentant un radical hydrocarboné aromatique cyclique ou bicyclique condensé.

3. Méthode selon la revendication 2, dans laquelle ladite base est choisie dans le groupe constitué par les amines tertiaires de formule N(R⁸)₃ ou les diamines tertiaires de formule N(R⁹)₃N, dans lesquelles R⁸ est un alkyle ayant deux à dix atomes de carbone ou un arylalkyle dont la partie alkyle comporte deux à dix atomes de carbone et R⁹ est un groupe de formule -(CH₂)ₙ- dans laquelle n va de deux à quatre, les amidines de formule R⁴C(NR⁵R⁶)=NR⁷ dans laquelle R⁵, R⁶ et R⁷ sont, chacun de façon indépendante, un alkyle comportant un à dix atomes de carbone et R⁴ est choisi parmi un amino et un alkyle comportant un à dix atomes de carbone ou dans laquelle R⁵ et/ou R⁶ considérés avec R⁴ et/ou R⁷ forment un groupe ayant pour formule -(CH₂)ₘ- dans laquelle m va de deux à six, les hydroxydes de formule M⁴OH, dans laquelle M⁴ est un cation approprié, les alcoolates de formule R³OM ou (R³O)₂M, dans lesquelles R³ est un alkyle comportant un à dix atomes de carbone et M est un cation approprié, les hydrures de formule M¹H, dans laquelle M¹ est un cation approprié, les carbonates de formule (M¹)₂CO₃ dans laquelle M¹ est un cation monovalent approprié ou de formule M²CO₃ dans laquelle M² est un cation divalent et les bicarbonates de formule M¹HCO₃ dans laquelle M¹ est un cation approprié.

4. Méthode selon la revendication 3, dans laquelle ladite base est le 1,8-diazabicyclo[5.4.0]undéc-7-ène (DBU) ou la 1,1,3,3-tétraméthylguanidine, R est -CH₂NO₂ ou -CH(NO₂)CH₃, X est fluoro, Y est nitro et Z est chloro.

5. Composé ayant pour formule 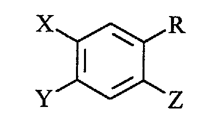 dans laquelle X est chloro ou fluoro, un parmi Y et Z est chloro et l'autre est nitro et R est un radical choisi dans le groupe constitué par -CCl₃, -CH₂NO₂, -CH(NO₂)R¹, -CH(CO₂R¹)₂, -CH(C(O)R²)₂, -CH(CN)CO₂R¹ et -CH(CO₂R¹)COR², dans lesquels R¹ représente un alkyle ou un arylalkyle et R² représente un alkyle, un aryle ou un arylalkyle et, où, s'ils apparaissent plus d'une fois dans un tel radical, R¹ et R² peuvent être, à chaque fois, identiques ou différents, le terme alkyle représentant un radical hydrocarboné saturé à chaîne droite ou ramifiée contenant un à dix atomes de carbone et le terme aryle représentant un radical hydrocarboné aromatique cyclique ou bicyclique condensé.
